# EUROPEAN PATENT APPLICATION

(11) **EP 4 129 982 A1**
(43) Date of publication of application: **08.02.2023**
(21) Application number: 21777018.9
(22) Date of filing: 26.03.2021
(51) Int. Cl.: C07D 211/78, A23L 33/125, A24D 3/06, A23G 4/06, A23L 19/00

(54) **ARECA NUT-FLAVORED PRODUCT**

(30) Priority: 27.03.2020 CN 202010227895; 24.06.2020 CN 202010587899
(71) Applicant: Shenzhen Icybetel Biological Technology Co., Ltd, Shenzhen, Guangdong 518000 (CN)
(72) Inventor: FU, Guofeng, Shenzhen, Guangdong 518000 (CN); LU, Jin, Shenzhen, Guangdong 518000 (CN); CHU, Ming, Shenzhen, Guangdong 518000 (CN); ZHOU, Xing, Shenzhen, Guangdong 518000 (CN); HUANG, Wenwen, Shenzhen, Guangdong 518000 (CN); XU, Zhongli, Shenzhen, Guangdong 518000 (CN); LI, Yonghai, Shenzhen, Guangdong 518000 (CN)
(74) Representative: Proi World Intellectual Property GmbH
(86) International application number: PCT/CN2021/083378
(87) International publication number: WO 2021/190643

(57) **Abstract**

This application provides an areca nut-flavored product, containing an arecoline salt. Because an arecoline salt is used in the product, a user absorbs arecoline when consuming the product, and an effect similar to chewing areca nuts is produced, with relatively little irritation.

## Description

### CROSS-REFERENCE TO RELATED APPLICATIONS

This application claims the priority to Chinese Patent Application No. 202010587899.7 filed with China National Intellectual Property Administration on June 24, 2020 and entitled "ARECA NUT-FLAVORED PRODUCT", and Chinese Patent Application No. 202010227895.8 filed with China National Intellectual Property Administration on March 27, 2020 and entitled "AEROSOL GENERATION SUBSTRATE", the contents of which are incorporated herein by reference in their entireties.

### TECHNICAL FIELD

This application belongs to the field of chemical synthesis technologies, and relates to an areca nut-flavored product.

### BACKGROUND

Arecoline, with a chemical name of "1,2,5,6-tetrahydro-1-methyl-3-pyridinecarboxylic acid methyl ester", is an oily liquid with a cholinergic effect. Arecoline is a compound with great application value, and preparation methods and applications thereof have always been concerned by people.

However, the properties of arecoline are unstable, and no product is commercially available at present. Therefore, it is impossible to determine the conversion rate of arecoline, and it is even more impossible to determine whether an effect similar to chewing areca nuts can be achieved. In addition, the application of an arecoline salt in products has not yet been found.

### SUMMARY

This application provides an areca nut-flavored product, containing an arecoline salt.

In some embodiments, the arecoline salt is obtained by neutralizing arecoline with a complex acid.

In some embodiments, the complex acid of the arecoline salt is selected from the group consisting of at least one of:
formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

In some embodiments, the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

In some embodiments, the product is an aerosol generation substrate configured to be atomized to generate a smokable aerosol.

In some embodiments, the arecoline salt has a mass percentage of 0.2-15%, preferably 1-15%, further preferably 2-15%, further preferably 2-10%, further preferably 2-5%, further preferably 2-4%, and more preferably 3-4%, based on total mass of the aerosol generation substrate.

In some embodiments, the arecoline salt is arecoline hydrobromide and/or arecoline benzoate.

In some embodiments, a method for preparing the arecoline salt includes the following steps: extracting arecoline from areca nuts by using ethyl acetate as an extractant, adjusting a pH value of the extract to 7.5-8.5, taking an ethyl acetate layer, adding hydrobromic acid or benzoic acid to generate an arecoline salt crystal, and recrystallizing the arecoline salt crystal to obtain arecoline hydrobromide or arecoline benzoate.

In some embodiments, the aerosol generation substrate further includes a solvent and a flavoring agent.

In some embodiments, the solvent is selected from the group consisting of at least one of:
propylene glycol, glycerol, polyethylene glycol 200, polyethylene glycol 400, dipropylene glycol ether, ethanol, water, triethyl citrate, triacetin, caprylic capric triglycerride, isopropyl alcohol, palm oil, peanut oil, corn oil or salad oil.

In some embodiments, the solvent includes glycerol, and the glycerol in the aerosol generation substrate has a mass percentage of 0-90%.

In some embodiments, the solvent includes propylene glycol, and the propylene glycol in the aerosol generation substrate has a mass percentage of 9-99.8%.

In some embodiments, the flavoring agent is selected from the group consisting of at least one of:
nerol, trans-2-hexenol, linalool, benzyl alcohol, 1-hexanol, leaf alcohol, alpha-terpineol, citronellol, beta-phenylethanol, linalool oxide, geraniol, isoamyl alcohol, octanol, hexanol, decanol, cinnamyl alcohol, heptanol, eugenol, maltol, ethyl maltol, thymol, isoeugenol, 2-methylbutyric acid, malic acid, n-valeric acid, n-hexanoic acid, edible acetic acid, n-caprylic acid, strawberry acid, butyric acid, citric acid, propionic acid, 3-methylvaleric acid, isovaleric acid, isoamyl acetate, amyl formate, geranyl formate, butyl formate, benzyl formate, formicacidhexenester, gamma-decanolactone, delta-nonalactone, gamma-octalactone, gammaheptalactone, gamma-Undecalactone, delta-dodecalactone, benzaldehyde, strawberry aldehyde, cinnamaldehyde, furfural, citral, acetaldehyde, 3-methylthiopropanal, natural 3-mercapto-2-methylpentanal, isobutyraldehyde, trans-2-octenal, trans-2-nonenal, trans-2-decenal, trans,trans-2,4-heptadienal, 2,5-dimethylpyrazine, 2-acetylfuran, 2-ethyl-3(5 or 6)-dimethylpyrazine, 2,3,5,6-tetramethylpyrazine, 2,3,5-trimethylpyrazine, 2-acetylpyrazine, acetophenone, beta-ionone, damascenone No. 2 (Firmenich), butanedione, alpha-ionone, acetoin (acetyl methyl carbinol), acetoin (acetyl methyl carbinol), methyl heptenone, vanillin, ethyl vanillin, dihydrocoumarin, raspberry ketone, anisole, cedrol methyl ether, methyl-2-methyl-3-furyl disulfide, wintergreen oil, clove bud oil, 10-fold orange oil, Bois de rose oil, geranium oil, benzaldehyde, basil oil, ethyl vanillin, dihydrocoumarin, raspberry ketone, vanitrope, Tolu concrete, Peru concrete, oak extract, supercritical extract from espresso (water soluble), cocoa extract, coffee tincture, pandan leaf extract, vanilla extract, Labdanum concrete, orris oil or concrete, jasmine concrete, tree moss concrete (amber), tamarind extract, Zimbabwean tobacco extract, tobacco essence, burley tobacco extract, flue-cured tobacco absolute A, top note extract of flue-cured tobacco or top note extract of sun-cured tobacco.

In some embodiments, the aerosol generation substrate is a liquid phase configured to be atomized by any one of a heating atomizer, an ultrasonic atomizer, an air compression atomizer or a press-type spraying device to generate an aerosol.

In some embodiments, the aerosol generation substrate is a solid phase configured to generate a smokable aerosol when heated below an ignition point.

In some embodiments, the product is a filter capsule used in an aerosol-generating article.

In some embodiments, the product is a chewing gum.

In some embodiments, the product is a beverage.

According to this application, because an arecoline salt is used in the product, a user absorbs arecoline when consuming the product, and an effect similar to chewing areca nuts is produced, with relatively little irritation.

### DESCRIPTION OF EMBODIMENTS

This application will be further described with reference to the following embodiments.

### Implementation 1

Implementation 1 of this application provides an application of the arecoline salt in preparing an aerosol generation substrate.

In some embodiments, the arecoline salt is obtained by neutralizing arecoline with a complex acid. In some embodiments, the complex acid of the arecoline salt is selected from the group consisting of at least one of: formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

In some embodiments, the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

In some embodiments, when the aerosol generation substrate is prepared, the arecoline salt has a mass percentage of 0.2-15%, preferably 1-15%, further preferably 2-15%, further preferably 2-10%, further preferably 2-5%, and further preferably 2-4%, based on total mass of the aerosol generation substrate.

In some embodiments, when the aerosol generation substrate is prepared, objects added to the arecoline salt include a solvent.

In some embodiments, the solvent is selected from the group consisting of at least one of:
1,2-propanediol, 1,3-propanediol, glycerol, polyethylene glycol 200, polyethylene glycol 400, dipropylene glycol ether, ethanol, water, triethyl citrate, triacetin, caprylic capric triglycerride, isopropyl alcohol, sweet orange oil, lemon oil, peppermint oil, palm oil, peanut oil, corn oil or salad oil.

In some embodiments, the solvent includes glycerol, and the glycerol has a mass percentage of 0-90%.

In some embodiments, the solvent includes propylene glycol, and the propylene glycol has a mass percentage of 9-99.8%.

In some embodiments, objects added to the arecoline salt further include a flavoring agent.

In some embodiments, the flavoring agent is selected from the group consisting of at least one of:
nerol, trans-2-hexenol, linalool, benzyl alcohol, 1-hexanol, leaf alcohol, alpha-terpineol, citronellol, beta-phenylethanol, linalool oxide, geraniol, isoamyl alcohol, octanol, hexanol, decanol, cinnamyl alcohol, heptanol, eugenol, maltol, ethyl maltol, thymol, isoeugenol, 2-methylbutyric acid, malic acid, n-valeric acid, n-hexanoic acid, edible acetic acid, n-caprylic acid, strawberry acid, butyric acid, citric acid, propionic acid, 3-methylvaleric acid, isovaleric acid, isoamyl acetate, amyl formate, geranyl formate, butyl formate, benzyl formate, formicacidhexenester, gamma-decanolactone, delta-nonalactone, gamma-octalactone, gammaheptalactone, gamma-Undecalactone, delta-dodecalactone, benzaldehyde, strawberry aldehyde, cinnamaldehyde, furfural, citral, acetaldehyde, 3-methylthiopropanal, natural 3-mercapto-2-methylpentanal, isobutyraldehyde, trans-2-octenal, trans-2-nonenal, trans-2-decenal, trans,trans-2,4-heptadienal, 2,5-dimethylpyrazine, 2-acetylfuran, 2-ethyl-3(5 or 6)-dimethylpyrazine, 2,3,5,6-tetramethylpyrazine, 2,3,5-trimethylpyrazine, 2-acetylpyrazine, acetophenone, beta-ionone, damascenone No. 2 (Firmenich), butanedione, alpha-ionone, acetoin (acetyl methyl carbinol), acetoin (acetyl methyl carbinol), methyl heptenone, vanillin, ethyl vanillin, dihydrocoumarin, raspberry ketone, anisole, cedrol methyl ether, methyl-2-methyl-3-furyl disulfide, wintergreen oil, clove bud oil, 10-fold orange oil, Bois de rose oil, geranium oil, benzaldehyde, basil oil, ethyl vanillin, dihydrocoumarin, raspberry ketone, vanitrope, Tolu concrete, Peru concrete, oak extract, supercritical extract from espresso (water soluble), cocoa extract, coffee tincture, pandan leaf extract, vanilla extract, Labdanum concrete, orris oil or concrete, jasmine concrete, tree moss concrete (amber), tamarind extract, Zimbabwean tobacco extract, tobacco essence, burley tobacco extract, flue-cured tobacco absolute A, top note extract of flue-cured tobacco or top note extract of sun-cured tobacco.

It should be noted that the flavoring agent is not limited to the substances listed above, and any flavoring agent conforming to FEMA codes or CAS codes is applicable.

In some embodiments, objects added to the arecoline salt do not include nicotine and/or nicotine salts.

### Implementation 2

Implementation 2 of this application provides an aerosol generation substrate including:
the arecoline salt;
a solvent; and
optionally a flavoring agent.

Preferably, the arecoline salt has a mass percentage of 0.2-15%, preferably 1-15%, further preferably 2-15%, further preferably 2-12%, further preferably 2-10%, further preferably 2-8%, further preferably 2-5%, further preferably 2-4%, further preferably 2-3%, and more preferably 3-4%, based on total mass of the aerosol generation substrate.

Preferably, the complex acid of the arecoline salt is selected from the group consisting of at least one of:
formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, benzoic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

Preferably, the arecoline salt is arecoline hydrobromide and/or arecoline benzoate.

Preferably, the arecoline salt is obtained by neutralizing arecoline extracted from areca nuts with the complex acid.

Preferably, the arecoline salt is arecoline hydrobromide or arecoline benzoate, and a method for preparing the arecoline salt includes the following steps: extracting arecoline from areca nuts by using ethyl acetate as an extractant, adjusting a pH value of the extract to 7.5-8.5, taking an ethyl acetate layer, adding hydrobromic acid or benzoic acid to generate an arecoline salt crystal, and recrystallizing the arecoline salt crystal to obtain arecoline hydrobromide or arecoline benzoate.

Preferably, the solvent is selected from the group consisting of at least one of:
propylene glycol, glycerol, polyethylene glycol 200, polyethylene glycol 400, dipropylene glycol ether, ethanol, water, triethyl citrate, triacetin, caprylic capric triglycerride, isopropyl alcohol, palm oil, peanut oil, corn oil or salad oil.

Preferably, the solvent includes glycerol, and the glycerol in the aerosol generation substrate has a mass percentage of 0-90%, preferably 0-80%, further preferably 0-70%, further preferably 0-60%, further preferably 10-60%, further preferably 20-60%, further preferably 30-60%, further preferably 40-60%, and more preferably 50-60%.

Preferably, the solvent includes propylene glycol, and the propylene glycol in the aerosol generation substrate has a mass percentage of 9-99.8%, preferably 9-90%, further preferably 9-80%, further preferably 9-70%, further preferably 9-60%, further preferably 9-50%, further preferably 20-50%, further preferably 30-50%, and more preferably 35-45%.

Preferably, the aerosol generation substrate includes a flavoring agent, and the flavoring agent is selected from the group consisting of at least one of:
nerol, trans-2-hexenol, linalool, benzyl alcohol, 1-hexanol, leaf alcohol, alpha-terpineol, citronellol, beta-phenylethanol, linalool oxide, geraniol, isoamyl alcohol, octanol, hexanol, decanol, cinnamyl alcohol, heptanol, eugenol, maltol, ethyl maltol, thymol, isoeugenol, 2-methylbutyric acid, malic acid, n-valeric acid, n-hexanoic acid, edible acetic acid, n-caprylic acid, strawberry acid, butyric acid, citric acid, propionic acid, 3-methylvaleric acid, isovaleric acid, isoamyl acetate, amyl formate, geranyl formate, butyl formate, benzyl formate, formicacidhexenester, gamma-decanolactone, delta-nonalactone, gamma-octalactone, gammaheptalactone, gamma-Undecalactone, delta-dodecalactone, benzaldehyde, strawberry aldehyde, cinnamaldehyde, furfural, citral, acetaldehyde, 3-methylthiopropanal, natural 3-mercapto-2-methylpentanal, isobutyraldehyde, trans-2-octenal, trans-2-nonenal, trans-2-decenal, trans,trans-2,4-heptadienal, 2,5-dimethylpyrazine, 2-acetylfuran, 2-ethyl-3(5 or 6)-dimethylpyrazine, 2,3,5,6-tetramethylpyrazine, 2,3,5-trimethylpyrazine, 2-acetylpyrazine, acetophenone, beta-ionone, damascenone No. 2 (Firmenich), butanedione, alpha-ionone, acetoin (acetyl methyl carbinol), acetoin (acetyl methyl carbinol), methyl heptenone, vanillin, ethyl vanillin, dihydrocoumarin, raspberry ketone, anisole, cedrol methyl ether, methyl-2-methyl-3-furyl disulfide, wintergreen oil, clove bud oil, 10-fold orange oil, Bois de rose oil, geranium oil, benzaldehyde, basil oil, ethyl vanillin, dihydrocoumarin, raspberry ketone, vanitrope, Tolu concrete, Peru concrete, oak extract, supercritical extract from espresso (water soluble), cocoa extract, coffee tincture, pandan leaf extract, vanilla extract, Labdanum concrete, orris oil or concrete, jasmine concrete, tree moss concrete (amber), tamarind extract, Zimbabwean tobacco extract, tobacco essence, burley tobacco extract, flue-cured tobacco absolute A, top note extract of flue-cured tobacco or top note extract of sun-cured tobacco.

It should be noted that the flavoring agent is not limited to the substances listed above, and any flavoring agent conforming to FEMA codes or CAS codes is applicable.

### Test method:

Arecoline hydrobromide is used as an arecoline salt, and a mixture of glycerol and propylene glycol at a mass ratio of 1:1 is selected as a solvent.

A smoking machine is used for smoking in accordance with international standards, and a Cambridge filter is used to collect an aerosol atomized from an aerosol generation substrate. The mass of the arecoline hydrobromide in the aerosol is tested to be m1. Then, the mass of the arecoline hydrobromide in the remaining aerosol generation substrate is tested to be m2, the mass of total arecoline hydrobromide in the aerosol generation substrate is m0 and the conversion rate k=m1/(m0-m2) is calculated.

For international standards involved, refer to:
CORESTA RECOMMENDED METHOD N°81, Afhor standardization XP D90-300-3, International Standard ISO 20768:2018 and PD CEN/TR 17236:2018.

Parameters for puffing an e-cigarette are as follows:

| | |
|---|---|
| Puff Duration | 3.0s±0.1s |
| Puff Volume | 55mL±0.3mL |
| Puff Frequency | 30s±0.5s |
| Puff of Each Group | 20 |
| Group Interval Time | 300s±120s |
| Maximum Flow | 18.5mL/s±0.1mL/s |
| Pressure Drop | <50hPa |
| Group | 5 |
| Total Number of Puff | 100 |
| Total Duration of Atomization | 300s |

### Test on puff experience

After aerosol generation substrates are atomized, puff experience of the aerosol generation substrates can be evaluated from the following two aspects: satisfaction of smokers after absorbing arecoline salts; and discomfort of the smokers after consuming too much arecoline salts. Different aerosol generation substrates are scored from 0 to 5 based on evaluation of multiple smokers and experience in terms of the above two aspects to obtain embodiments with better puff experience.

### Test results

| Embodiment | Arecoline salt content (wt%) | k | Arecoline salt content of aerosol (wt%) | Puff experience |
|---|---|---|---|---|
| 1 | Arecoline hydrobromide (0.1%) | 89% | 0.09% | 0 |
| 2 | Arecoline hydrobromide (0.2%) | 87% | 0.17% | 1 |
| 3 | Arecoline hydrobromide (1%) | 79% | 0.8% | 1 |
| 4 | Arecoline hydrobromide (2%) | 77% | 1.5% | 2 |
| 5 | Arecoline hydrobromide (3%) | 75% | 2.2% | 5 |
| 6 | Arecoline hydrobromide (4%) | 68% | 2.7% | 4 |
| 7 | Arecoline hydrobromide (5%) | 62% | 3.1% | 3 |
| 8 | Arecoline hydrobromide (10%) | 56% | 5.6% | 2 |
| 9 | Arecoline hydrobromide (15%) | 56% | 8.4% | 1 |
| 10 | Arecoline hydrobromide (20%) | 45% | 9.0% | 0 |

It is clear from the test results that the conversion rate decreases with the increase of the arecoline hydrobromide content of the aerosol generation substrates, and the conversion rate is still higher than 50% when the arecoline hydrobromide content of the aerosol generation substrates reaches 15%. In addition, after the aerosol generation substrates with different arecoline hydrobromide content are atomized into aerosols, the arecoline salt content of the aerosols is different, and therefore, smokers have different experience. When the arecoline salt content of the aerosol generation substrates is less than 0.2%, smokers cannot feel the satisfaction brought by the intake of arecoline. The satisfaction increases with the increase of the content. After the arecoline salt content is more than 5%, discomfort caused by the intake of arecoline salts begin to appear, and gradually appears with the increase of the content under experimental conditions. When the content is more than 15%, the discomfort is unacceptable.

### Implementation 3

Implementation 3 of this application provides an aerosol generating system including a atomizer and the aerosol generation substrate according to implementation 2;
the atomizer is configured to atomize the aerosol generation substrate to form an aerosol.

In some embodiments, the atomizer is a heating atomizer.

In some embodiments, the atomizer is an ultrasonic atomizer.

In some embodiments, the aerosol generation substrate includes an arecoline salt, glycerol and propylene glycol.

In some embodiments, the ultrasonic atomizer has an oscillation frequency greater than 1 MHz.

In some embodiments, the atomizer is an air compression atomizer or a press-type spraying device.

In some embodiments, the aerosol generation substrate includes an arecoline salt, ethyl alcohol and water.

### Implementation 4

Implementation 4 of this application provides an application of the arecoline salt in manufacturing an aerosol-generating article.

In some embodiments, the arecoline salt is obtained by neutralizing arecoline with a complex acid. In some embodiments, the complex acid of the arecoline salt is selected from the group consisting of at least one of: formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

In some embodiments, the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

In some embodiments, when the aerosol-generating article is manufactured, the arecoline salt has a mass percentage of 0.2-15%, preferably 1-15%, further preferably 2-15%, further preferably 2-10%, further preferably 2-5%, further preferably 2-4%, and more preferably 3-4%, based on total mass of raw materials of the aerosol-generating article.

In some embodiments, when the aerosol-generating article is manufactured, objects added to the arecoline salt include an atomizing agent, an adhesive and a plant fiber.

In some embodiments, when the aerosol-generating article is manufactured, the atomizing agent has a mass percentage of 5-30%, preferably 10-30%, further preferably 10-25%, and further preferably 15-25%, based on total mass of raw materials of the aerosol-generating article.

In some embodiments, the atomizing agent is selected from the group consisting of at least one of:
1,2-propanediol, 1,3-propanediol, glycerol, polyethylene glycol 200, polyethylene glycol 400, dipropylene glycol ether, ethanol, water, triethyl citrate, triacetin, caprylic capric triglycerride, isopropyl alcohol, sweet orange oil, lemon oil, peppermint oil, palm oil, peanut oil, corn oil or salad oil.

In some embodiments, when the aerosol-generating article is manufactured, the adhesive has a mass percentage of 1-20%, preferably 1-15%, further preferably 1-10%, further preferably 3-10%, and further preferably 5-10%, based on total mass of raw materials of the aerosol-generating article.

In some embodiments, the adhesive is selected from the group consisting of at least one of:
carboxymethyl cellulose, hydroxypropyl methyl cellulose, polyvinyl alcohol, rosin, xanthan gum, Arabic gum, guar gum, chitosan, pectin, locust bean gum, starch, sodium alginate or shellac.

In some embodiments, when the aerosol-generating article is manufactured, the plant fiber has a mass percentage of 40-80%, preferably 40-70%, further preferably 50-70%, further preferably 50-65%, and further preferably 50-60%, based on total mass of raw materials of the aerosol-generating article.

In some embodiments, the plant fiber is selected from the group consisting of at least one of:
areca nut fiber, wood pulp fiber, hemp pulp fiber, cotton pulp fiber, bagasse pulp fiber, bamboo pulp fiber or coconut pulp fiber.

In some embodiments, objects added to the arecoline salt further include a flavoring agent.

In some embodiments, the flavoring agent is selected from the group consisting of at least one of:
nerol, trans-2-hexenol, linalool, benzyl alcohol, 1-hexanol, leaf alcohol, alpha-terpineol, citronellol, beta-phenylethanol, linalool oxide, geraniol, isoamyl alcohol, octanol, hexanol, decanol, cinnamyl alcohol, heptanol, eugenol, maltol, ethyl maltol, thymol, isoeugenol, 2-methylbutyric acid, malic acid, n-valeric acid, n-hexanoic acid, edible acetic acid, n-caprylic acid, strawberry acid, butyric acid, citric acid, propionic acid, 3-methylvaleric acid, isovaleric acid, isoamyl acetate, amyl formate, geranyl formate, butyl formate, benzyl formate, formicacidhexenester, gamma-decanolactone, delta-nonalactone, gamma-octalactone, gammaheptalactone, gamma-Undecalactone, delta-dodecalactone, benzaldehyde, strawberry aldehyde, cinnamaldehyde, furfural, citral, acetaldehyde, 3-methylthiopropanal, natural 3-mercapto-2-methylpentanal, isobutyraldehyde, trans-2-octenal, trans-2-nonenal, trans-2-decenal, trans,trans-2,4-heptadienal, 2,5-dimethylpyrazine, 2-acetylfuran, 2-ethyl-3(5 or 6)-dimethylpyrazine, 2,3,5,6-tetramethylpyrazine, 2,3,5-trimethylpyrazine, 2-acetylpyrazine, acetophenone, beta-ionone, damascenone No. 2 (Firmenich), butanedione, alpha-ionone, acetoin (acetyl methyl carbinol), acetoin (acetyl methyl carbinol), methyl heptenone, vanillin, ethyl vanillin, dihydrocoumarin, raspberry ketone, anisole, cedrol methyl ether, methyl-2-methyl-3-furyl disulfide, wintergreen oil, clove bud oil, 10-fold orange oil, Bois de rose oil, geranium oil, benzaldehyde, basil oil, ethyl vanillin, dihydrocoumarin, raspberry ketone, vanitrope, Tolu concrete, Peru concrete, oak extract, supercritical extract from espresso (water soluble), cocoa extract, coffee tincture, pandan leaf extract, vanilla extract, Labdanum concrete, orris oil or concrete, jasmine concrete, tree moss concrete (amber), tamarind extract, Zimbabwean tobacco extract, tobacco essence, burley tobacco extract, flue-cured tobacco absolute A, top note extract of flue-cured tobacco or top note extract of sun-cured tobacco.

It should be noted that the flavoring agent is not limited to the substances listed above, and any flavoring agent conforming to FEMA codes or CAS codes is applicable.

In some embodiments, objects added to the arecoline salt do not include tobacco components.

In some embodiments, a method for manufacturing the aerosol-generating article includes one of paper-making process, dry paper-making process, slurry process and roller-formation process.

### Implementation 5

Implementation 5 of this application provides an aerosol-generating article obtained from raw materials including the arecoline salt, an atomizing agent, an adhesive and a plant fiber.

In some embodiments, the arecoline salt is obtained by neutralizing arecoline with a complex acid. In some embodiments, the complex acid of the arecoline salt is selected from the group consisting of at least one of: formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

In some embodiments, the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

In some embodiments, the arecoline salt has a mass percentage of 0.2-15%, preferably 1-15%, further preferably 2-15%, further preferably 2-10%, further preferably 2-5%, further preferably 2-4%, and more preferably 3-4%, based on total mass of the raw materials.

In some embodiments, the atomizing agent has a mass percentage of 5-30%, preferably 10-30%, further preferably 10-25%, and further preferably 15-25%, based on total mass of the raw materials.

In some embodiments, the atomizing agent is selected from the group consisting of at least one of:
1,2-propanediol, 1,3-propanediol, glycerol, polyethylene glycol 200, polyethylene glycol 400, dipropylene glycol ether, ethanol, water, triethyl citrate, triacetin, caprylic capric triglycerride, isopropyl alcohol, sweet orange oil, lemon oil, peppermint oil, palm oil, peanut oil, corn oil or salad oil.

In some embodiments, the adhesive has a mass percentage of 1-20%, preferably 1-15%, further preferably 1-10%, further preferably 3-10%, and further preferably 5-10%, based on total mass of the raw materials.

In some embodiments, the adhesive is selected from the group consisting of at least one of:
carboxymethyl cellulose, hydroxypropyl methyl cellulose, polyvinyl alcohol, rosin, xanthan gum, Arabic gum, guar gum, chitosan, pectin, locust bean gum, starch, sodium alginate or shellac.

In some embodiments, the plant fiber has a mass percentage of 40-80%, preferably 40-70%, further preferably 50-70%, further preferably 50-65%, and further preferably 50-60%, based on total mass of the raw materials.

In some embodiments, the plant fiber is selected from the group consisting of at least one of:
areca nut fiber, wood pulp fiber, hemp pulp fiber, cotton pulp fiber, bagasse pulp fiber, bamboo pulp fiber or coconut pulp fiber.

In some embodiments, the raw materials further include a flavoring agent.

In some embodiments, the flavoring agent is selected from the group consisting of at least one of:
nerol, trans-2-hexenol, linalool, benzyl alcohol, 1-hexanol, leaf alcohol, alpha-terpineol, citronellol, beta-phenylethanol, linalool oxide, geraniol, isoamyl alcohol, octanol, hexanol, decanol, cinnamyl alcohol, heptanol, eugenol, maltol, ethyl maltol, thymol, isoeugenol, 2-methylbutyric acid, malic acid, n-valeric acid, n-hexanoic acid, edible acetic acid, n-caprylic acid, strawberry acid, butyric acid, citric acid, propionic acid, 3-methylvaleric acid, isovaleric acid, isoamyl acetate, amyl formate, geranyl formate, butyl formate, benzyl formate, formicacidhexenester, gamma-decanolactone, delta-nonalactone, gamma-octalactone, gammaheptalactone, gamma-Undecalactone, delta-dodecalactone, benzaldehyde, strawberry aldehyde, cinnamaldehyde, furfural, citral, acetaldehyde, 3-methylthiopropanal, natural 3-mercapto-2-methylpentanal, isobutyraldehyde, trans-2-octenal, trans-2-nonenal, trans-2-decenal, trans,trans-2,4-heptadienal, 2,5-dimethylpyrazine, 2-acetylfuran, 2-ethyl-3(5 or 6)-dimethylpyrazine, 2,3,5,6-tetramethylpyrazine, 2,3,5-trimethylpyrazine, 2-acetylpyrazine, acetophenone, beta-ionone, damascenone No. 2 (Firmenich), butanedione, alpha-ionone, acetoin (acetyl methyl carbinol), acetoin (acetyl methyl carbinol), methyl heptenone, vanillin, ethyl vanillin, dihydrocoumarin, raspberry ketone, anisole, cedrol methyl ether, methyl-2-methyl-3-furyl disulfide, wintergreen oil, clove bud oil, 10-fold orange oil, Bois de rose oil, geranium oil, benzaldehyde, basil oil, ethyl vanillin, dihydrocoumarin, raspberry ketone, vanitrope, Tolu concrete, Peru concrete, oak extract, supercritical extract from espresso (water soluble), cocoa extract, coffee tincture, pandan leaf extract, vanilla extract, Labdanum concrete, orris oil or concrete, jasmine concrete, tree moss concrete (amber), tamarind extract, Zimbabwean tobacco extract, tobacco essence, burley tobacco extract, flue-cured tobacco absolute A, top note extract of flue-cured tobacco or top note extract of sun-cured tobacco.

It should be noted that the flavoring agent is not limited to the substances listed above, and any flavoring agent conforming to FEMA codes or CAS codes is applicable.

In some embodiments, the raw materials do not include tobacco components.

### Embodiment 1

A heat-not-burn cigarette is prepared according to the following steps:
Step 1: preparation of a sheet substrate. Solid residues of an areca nut extract are selected and soaked in water at 60-80°C for 0.5-2h before filtering, the filtered solid is pulped to obtain a slurry, then 50-70 parts of slurry and 3-10 parts of solid adhesive are measured by weight and mixed well, and then a resulting mixed slurry is sent to a paper machine for molding and drying until the moisture content is about 10-15% to obtain a sheet substrate;
   the solid adhesive may be one or more of carboxymethyl cellulose, hydroxypropyl methyl cellulose, polyvinyl alcohol, rosin, xanthan gum, Arabic gum, guar gum, chitosan, pectin, locust bean gum, starch, sodium alginate or shellac.
Step 2: preparation of a coating fluid. 1-50 parts of arecoline salt, 2-20 parts of perfume and 40-80 parts of atomizing agent are measured by weight, and mixed well to obtain a coating fluid;
   the atomizing agent is a mixture of glycerol and propylene glycol at a mixing ratio of3-5: 1.
Step 3: coating of the sheet substrate. The coating fluid obtained in the step 2 is weighed at 25-40% of the weight of the sheet substrate obtained in the step 1, then sprayed onto the sheet substrate, and allowed to stand under a constant temperature and a constant humidity for 40-60h to obtain a plant sheet containing the arecoline salt.
Step 4: preparation of a heat-not-burn cigarette. The plant sheet obtained in the step 3 is made into a cigarette stick by size, and the cigarette stick is connected to a filter tip to obtain a heat-not-burn cigarette containing the arecoline salt.

The heat-not-burn cigarette prepared by the above preparation method is heated by an aerosol generation apparatus (by means of resistance heating), and surface temperature of a heating element in a working state is tested by an infrared thermal imager. Test results are shown in the following table.

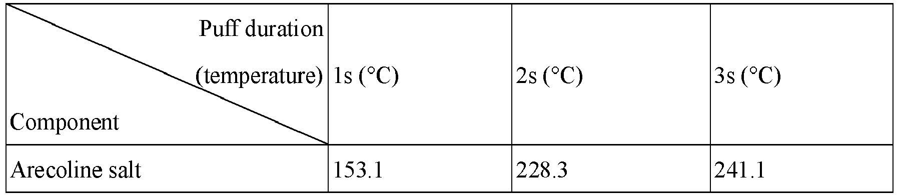

It is clear from the test results that the arecoline salt in the cigarette can be atomized at 150-250°C and generate a smokable aerosol.

The heat-not-burn cigarette prepared by the above preparation method is compared with an ordinary heat-not-burn cigarette for smoking and sensory quality evaluation: arecoline can be taken in from the heat-not-burn cigarette prepared by the above preparation method, and an effect similar to chewing areca nuts is produced, with relatively little irritation.

### Implementation 6

Implementation 6 of this application provides an application of the arecoline salt in preparing a liquid composition for a filter capsule; where the filter capsule includes a fragile shell and the liquid composition in the fragile shell.

In some embodiments, the arecoline salt is obtained by neutralizing arecoline with a complex acid.

In some embodiments, the complex acid of the arecoline salt is selected from the group consisting of at least one of:
formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

In some embodiments, the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

In some embodiments, the arecoline salt has a mass percentage of 0.2-15%, preferably 1-15%, further preferably 2-15%, further preferably 2-10%, further preferably 2-5%, further preferably 2-4%, and more preferably 3-4%, based on total mass of the liquid composition.

In some embodiments, when the liquid composition is prepared, objects added to the arecoline salt include an atomizing agent.

In some embodiments, the atomizing agent has a mass percentage of 5-30%, preferably 10-30%, further preferably 10-25%, and further preferably 15-25%, based on total mass of the liquid composition.

In some embodiments, the atomizing agent is selected from the group consisting of at least one of:
1,2-propanediol, 1,3-propanediol, glycerol, polyethylene glycol 200, polyethylene glycol 400, dipropylene glycol ether, ethanol, water, triethyl citrate, triacetin, caprylic capric triglycerride, isopropyl alcohol, sweet orange oil, lemon oil, peppermint oil, palm oil, peanut oil, corn oil or salad oil.

In some embodiments, objects added to the arecoline salt further include a flavoring agent.

In some embodiments, the flavoring agent is selected from the group consisting of at least one of:
nerol, trans-2-hexenol, linalool, benzyl alcohol, 1-hexanol, leaf alcohol, alpha-terpineol, citronellol, beta-phenylethanol, linalool oxide, geraniol, isoamyl alcohol, octanol, hexanol, decanol, cinnamyl alcohol, heptanol, eugenol, maltol, ethyl maltol, thymol, isoeugenol, 2-methylbutyric acid, malic acid, n-valeric acid, n-hexanoic acid, edible acetic acid, n-caprylic acid, strawberry acid, butyric acid, citric acid, propionic acid, 3-methylvaleric acid, isovaleric acid, isoamyl acetate, amyl formate, geranyl formate, butyl formate, benzyl formate, formicacidhexenester, gamma-decanolactone, delta-nonalactone, gamma-octalactone, gammaheptalactone, gamma-Undecalactone, delta-dodecalactone, benzaldehyde, strawberry aldehyde, cinnamaldehyde, furfural, citral, acetaldehyde, 3-methylthiopropanal, natural 3-mercapto-2-methylpentanal, isobutyraldehyde, trans-2-octenal, trans-2-nonenal, trans-2-decenal, trans,trans-2,4-heptadienal, 2,5-dimethylpyrazine, 2-acetylfuran, 2-ethyl-3(5 or 6)-dimethylpyrazine, 2,3,5,6-tetramethylpyrazine, 2,3,5-trimethylpyrazine, 2-acetylpyrazine, acetophenone, beta-ionone, damascenone No. 2 (Firmenich), butanedione, alpha-ionone, acetoin (acetyl methyl carbinol), acetoin (acetyl methyl carbinol), methyl heptenone, vanillin, ethyl vanillin, dihydrocoumarin, raspberry ketone, anisole, cedrol methyl ether, methyl-2-methyl-3-furyl disulfide, wintergreen oil, clove bud oil, 10-fold orange oil, Bois de rose oil, geranium oil, benzaldehyde, basil oil, ethyl vanillin, dihydrocoumarin, raspberry ketone, vanitrope, Tolu concrete, Peru concrete, oak extract, supercritical extract from espresso (water soluble), cocoa extract, coffee tincture, pandan leaf extract, vanilla extract, Labdanum concrete, orris oil or concrete, jasmine concrete, tree moss concrete (amber), tamarind extract, Zimbabwean tobacco extract, tobacco essence, burley tobacco extract, flue-cured tobacco absolute A, top note extract of flue-cured tobacco or top note extract of sun-cured tobacco.

It should be noted that the flavoring agent is not limited to the substances listed above, and any flavoring agent conforming to FEMA codes or CAS codes is applicable.

In some embodiments, objects added to the arecoline salt do not include nicotine and/or nicotine salts.

In some embodiments, the filter capsule is implanted in the aerosol-generating article.

In some embodiments, raw materials of the aerosol-generating article do not include tobacco components.

### Implementation 7

Implementation 7 of this application provides a filter capsule including a fragile shell and a liquid composition in the fragile shell;
the liquid composition includes the arecoline salt and an atomizing agent.

In some embodiments, the arecoline salt is obtained by neutralizing arecoline with a complex acid. In some embodiments, the complex acid of the arecoline salt is selected from the group consisting of at least one of: formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

In some embodiments, the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

In some embodiments, the arecoline salt has a mass percentage of 0.2-15%, preferably 1-15%, further preferably 2-15%, further preferably 2-10%, further preferably 2-5%, further preferably 2-4%, and more preferably 3-4%, based on total mass of the liquid composition.

In some embodiments, the atomizing agent has a mass percentage of 5-30%, preferably 10-30%, further preferably 10-25%, and further preferably 15-25%, based on total mass of the liquid composition.

In some embodiments, the atomizing agent is selected from the group consisting of at least one of:
1,2-propanediol, 1,3-propanediol, glycerol, polyethylene glycol 200, polyethylene glycol 400, dipropylene glycol ether, ethanol, water, triethyl citrate, triacetin, caprylic capric triglycerride, isopropyl alcohol, sweet orange oil, lemon oil, peppermint oil, palm oil, peanut oil, corn oil or salad oil.

In some embodiments, the liquid composition further includes a flavoring agent.

In some embodiments, the flavoring agent is selected from the group consisting of at least one of:
nerol, trans-2-hexenol, linalool, benzyl alcohol, 1-hexanol, leaf alcohol, alpha-terpineol, citronellol, beta-phenylethanol, linalool oxide, geraniol, isoamyl alcohol, octanol, hexanol, decanol, cinnamyl alcohol, heptanol, eugenol, maltol, ethyl maltol, thymol, isoeugenol, 2-methylbutyric acid, malic acid, n-valeric acid, n-hexanoic acid, edible acetic acid, n-caprylic acid, strawberry acid, butyric acid, citric acid, propionic acid, 3-methylvaleric acid, isovaleric acid, isoamyl acetate, amyl formate, geranyl formate, butyl formate, benzyl formate, formicacidhexenester, gamma-decanolactone, delta-nonalactone, gamma-octalactone, gammaheptalactone, gamma-Undecalactone, delta-dodecalactone, benzaldehyde, strawberry aldehyde, cinnamaldehyde, furfural, citral, acetaldehyde, 3-methylthiopropanal, natural 3-mercapto-2-methylpentanal, isobutyraldehyde, trans-2-octenal, trans-2-nonenal, trans-2-decenal, trans,trans-2,4-heptadienal, 2,5-dimethylpyrazine, 2-acetylfuran, 2-ethyl-3(5 or 6)-dimethylpyrazine, 2,3,5,6-tetramethylpyrazine, 2,3,5-trimethylpyrazine, 2-acetylpyrazine, acetophenone, beta-ionone, damascenone No. 2 (Firmenich), butanedione, alpha-ionone, acetoin (acetyl methyl carbinol), acetoin (acetyl methyl carbinol), methyl heptenone, vanillin, ethyl vanillin, dihydrocoumarin, raspberry ketone, anisole, cedrol methyl ether, methyl-2-methyl-3-furyl disulfide, wintergreen oil, clove bud oil, 10-fold orange oil, Bois de rose oil, geranium oil, benzaldehyde, basil oil, ethyl vanillin, dihydrocoumarin, raspberry ketone, vanitrope, Tolu concrete, Peru concrete, oak extract, supercritical extract from espresso (water soluble), cocoa extract, coffee tincture, pandan leaf extract, vanilla extract, Labdanum concrete, orris oil or concrete, jasmine concrete, tree moss concrete (amber), tamarind extract, Zimbabwean tobacco extract, tobacco essence, burley tobacco extract, flue-cured tobacco absolute A, top note extract of flue-cured tobacco or top note extract of sun-cured tobacco.

It should be noted that the flavoring agent is not limited to the substances listed above, and any flavoring agent conforming to FEMA codes or CAS codes is applicable.

In some embodiments, the liquid composition does not include nicotine and/or nicotine salts.

### Implementation 8

Implementation 8 of this application provides an aerosol-generating article including the filter capsule according to Implementation 7.

In some embodiments, raw materials of the aerosol-generating article do not include tobacco components.

In the implementation, the aerosol-generating article can be prepared by a paper-making process, a dry paper-making process, a slurry process or a roller-formation process. Specifically, refer to the contents of the above implementations for raw materials and preparation steps of the aerosol-generating article.

A filter capsule can be implanted into a filter tip section (not limited to this position) of a heat-not-burn cigarette by embedding the filter capsule in a filter stick (not limited to this technology). When in use, a smoker can break the filter capsule to take in substances such as arecoline.

It should be noted that in the implementation, there is no special restriction on components and preparation processes of the fragile shell, as well as preparation processes of the filter capsule, and techniques well known to those skilled in the art can be used.

### Embodiment 1

A liquid composition in a filter capsule is prepared according to the following formula:
10wt% arecoline salt, 80wt% caprylic capric triglycerride and 10wt% menthol.

Preparation method: arecoline salt of the above formula weight is weighed, then caprylic capric triglycerride and menthol are added under stirring, and mixed well to obtain the liquid composition.

The heat-not-burn cigarette containing the filter capsule according to Embodiment 1 is compared with a heat-not-burn cigarette without a filter capsule for smoking and sensory quality evaluation: arecoline can be taken in from the heat-not-burn cigarette containing the filter capsule according to Embodiment 1, and an effect similar to chewing areca nuts is produced, with relatively little irritation.

### Implementation 9

Implementation 9 of this application provides an application of the arecoline salt in manufacturing a chewing gum.

In some embodiments, the arecoline salt is obtained by neutralizing arecoline with a complex acid. In some embodiments, the complex acid of the arecoline salt is selected from the group consisting of at least one of: formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

In some embodiments, the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

In some embodiments, when the chewing gum is manufactured, the arecoline salt has a mass percentage of 0.02-2%, preferably 0.02-1.6%, further preferably 0.02-1.2%, further preferably 0.02-1%, further preferably 0.1-1%, further preferably 0.2-1%, and further preferably 0.4-1%, based on total mass of raw materials of the chewing gum.

In some embodiments, when the chewing gum is manufactured, objects added to the arecoline salt include a gum base.

In some embodiments, when the chewing gum is manufactured, the gum base has a mass percentage of 20-75%, preferably 20-70%, further preferably 20-60%, further preferably 20-50%, further preferably 25-50%, further preferably 30-50%, further preferably 35-50%, and further preferably 35-45%, based on total mass of raw materials of the chewing gum.

In some embodiments, objects added to the arecoline salt further include a sweetening agent.

In some embodiments, the sweetening agent is selected from the group consisting of at least one of:
white granulated sugar, rock sugar, sucrose, maltose, corn syrup, glucose syrup, fructose, maltodextrin, polydextrose, xylitol, sorbitol, maltitol, erythritol, aspartame, acesulfame, sucralose, stevioside or neotame.

In some embodiments, when the chewing gum is manufactured, the sweetening agent has a mass percentage of 20-75%, preferably 20-70%, further preferably 20-60%, further preferably 20-50%, further preferably 25-50%, further preferably 25-40%, further preferably 25-35%, and further preferably 30-35%, based on total mass of raw materials of the chewing gum.

In some embodiments, objects added to the arecoline salt further include a filler material.

In some embodiments, the filler material is selected from the group consisting of at least one of:
sodium carbonate, sodium bicarbonate, potassium carbonate, calcium carbonate, dicalcium phosphate or sodium dihydrogen phosphate.

In some embodiments, when the chewing gum is manufactured, the filler material has a mass percentage of 0.03-23%, preferably 0.03-20%, further preferably 0.03-15%, further preferably 0.03-10%, further preferably 0.5-10%, further preferably 1-10%, further preferably 1.5-10%, further preferably 2-8%, and further preferably 2-6%, based on total mass of raw materials of the chewing gum.

In some embodiments, objects added to the arecoline salt further include a flavor.

In some embodiments, the flavor is selected from the group consisting of at least one of:
mint, spearmint, cinnamon, borneol or essence.

### Implementation 10

Implementation 10 of this application provides a chewing gum obtained from raw materials including the arecoline salt and a gum base.

In some embodiments, the arecoline salt is obtained by neutralizing arecoline with a complex acid. In some embodiments, the complex acid of the arecoline salt is selected from the group consisting of at least one of: formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

In some embodiments, the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

In some embodiments, the arecoline salt has a mass percentage of 0.02-2%, preferably 0.02-1.6%, further preferably 0.02-1.2%, further preferably 0.02-1%, further preferably 0.1-1%, further preferably 0.2-1%, and further preferably 0.4-1%, based on total mass of the raw materials.

In some embodiments, the gum base has a mass percentage of 20-75%, preferably 20-70%, further preferably 20-60%, further preferably 20-50%, further preferably 25-50%, further preferably 30-50%, further preferably 35-50%, and further preferably 35-45%, based on total mass of the raw materials.

In some embodiments, the raw materials further include a sweetening agent.

In some embodiments, the sweetening agent is selected from the group consisting of at least one of:
white granulated sugar, rock sugar, sucrose, maltose, corn syrup, glucose syrup, fructose, maltodextrin, polydextrose, xylitol, sorbitol, maltitol, erythritol, aspartame, acesulfame, sucralose, stevioside or neotame.

In some embodiments, the sweetening agent has a mass percentage of 20-75%, preferably 20-70%, further preferably 20-60%, further preferably 20-50%, further preferably 25-50%, further preferably 25-40%, further preferably 25-35%, and further preferably 30-35%, based on total mass of the raw materials.

In some embodiments, the raw materials further include a filler material.

In some embodiments, the filler material is selected from the group consisting of at least one of:
sodium carbonate, sodium bicarbonate, potassium carbonate, calcium carbonate, dicalcium phosphate or sodium dihydrogen phosphate.

In some embodiments, the filler material has a mass percentage of 0.03-23%, preferably 0.03-20%, further preferably 0.03-15%, further preferably 0.03-10%, further preferably 0.5-10%, further preferably 1-10%, further preferably 1.5-10%, further preferably 2-8%, and further preferably 2-6%, based on total mass of the raw materials.

In some embodiments, the raw materials further include a flavor.

In some embodiments, the flavor is selected from the group consisting of at least one of:
mint, spearmint, cinnamon, borneol or essence.

### Embodiment 1

A chewing gum is prepared according to the following formula:
54wt% gum base, 23wt% powdered sugar, 14wt% glucose syrup, 4wt% potassium carbonate, 3wt% peppermint flavor, 1wt% menthol and 1wt% arecoline salt.

Preparation method: a gum base is heated to 70-105°C and stirred in a blender, then powdered sugar is added while allowing to cool to 60-80°C, then a buffer pair, additives, glucose syrup and an arecoline salt are added for finally rolling and molding to obtain a finished product.

### Embodiment 2

A xylitol chewing gum is prepared according to the following formula:
54wt% gum base, 23wt% xylitol, 14wt% non-sucrose, 4wt% potassium carbonate, 3wt% peppermint flavor, 1wt% menthol and 1wt% arecoline salt.

Preparation method: a gum base is heated to 70-105°C and stirred in a blender, then xylitol and non-sucrose are added while allowing to cool to 60-80°C, then a buffer pair, additives and an arecoline salt are added for finally rolling and molding to obtain a finished product.

The chewing gums prepared by the above preparation methods are compared with an ordinary chewing gum for edible and sensory quality evaluation: the chewing gums prepared by the above preparation methods can produce an effect similar to chewing areca nuts, with relatively little irritation and good taste. Because the chewing gums do not contain lime powder and cellulose, the problems of abrasion and irritation of oral mucosa and induction of oral diseases when chewing areca nuts are avoided.

### Implementation 11

Implementation 11 of this application provides an application of the arecoline salt in preparing a beverage.

In some embodiments, the arecoline salt is obtained by neutralizing arecoline with a complex acid. In some embodiments, the complex acid of the arecoline salt is selected from the group consisting of at least one of: formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

In some embodiments, the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

In some embodiments, when the beverage is prepared, the arecoline salt has a mass percentage of 0.01-0.5%, preferably 0.01-0.4%, further preferably 0.01-0.3%, further preferably 0.01-0.2%, and further preferably 0.05-0.2%, based on total mass of the beverage.

In some embodiments, when the beverage is prepared, objects added to the arecoline salt include water.

In some embodiments, when the beverage is prepared, the water has a mass percentage of 75-90%, based on total mass of the beverage.

In some embodiments, when the beverage is prepared, objects added to the arecoline salt further include a functional component.

In some embodiments, the functional component is selected from the group consisting of at least one of:
concentrated momordica grosvenori juice, natural menthol, concentrated liquorice juice, concentrated honeysuckle juice, chrysanthemum extract, kudzuvine root extract and loquat puree.

In some embodiments, the functional component has a mass percentage of 0.5-2%, based on total mass of the beverage.

In some embodiments, when the beverage is prepared, objects added to the arecoline salt further include an acidity regulator.

In some embodiments, the acidity regulator is selected from the group consisting of at least one of:
tartaric acid, malic acid, citric acid and sodium citrate.

In some embodiments, when the beverage is prepared, the acidity regulator has a mass percentage of 0.1-5%, based on total mass of the beverage.

In some embodiments, when the beverage is prepared, objects added to the arecoline salt further include a sweetening agent.

In some embodiments, the sweetening agent is selected from the group consisting of at least one of:
white granulated sugar, rock sugar, sucrose, maltose, corn syrup, glucose syrup, fructose, maltodextrin, polydextrose, xylitol, sorbitol, maltitol, erythritol, aspartame, acesulfame, sucralose, stevioside or neotame.

In some embodiments, when the beverage is prepared, the sweetening agent has a mass percentage of 0.5-5%, based on total mass of the beverage.

In some embodiments, when the beverage is prepared, objects added to the arecoline salt further include an antioxidant.

In some embodiments, the antioxidant is selected from the group consisting of at least one of:
ascorbic acid, sodium erythorbate, vitamin C and vitamin E.

In some embodiments, when the beverage is prepared, the antioxidant has a mass percentage of 0.05-0.15%, based on total mass of the beverage.

In some embodiments, when the beverage is prepared, objects added to the arecoline salt further include a preservative.

In some embodiments, the preservative is selected from the group consisting of at least one of:
sodium benzoate, sodium sorbate, and potassium sorbate.

In some embodiments, when the beverage is prepared, the preservative has a mass percentage of 0.01-0.05%, based on total mass of the beverage.

In some embodiments, when the beverage is prepared, objects added to the arecoline salt further include an edible essence.

In some embodiments, when the beverage is prepared, the edible essence has a mass percentage of 0.5-5%, based on total mass of the beverage.

### Implementation 12

Implementation 12 of this application provides a beverage obtained from raw materials including the arecoline salt and water. In some embodiments, the arecoline salt is obtained by neutralizing arecoline with a complex acid. In some embodiments, the complex acid of the arecoline salt is selected from the group consisting of at least one of: formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

In some embodiments, the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

In some embodiments, the arecoline salt has a mass percentage of 0.01-0.5%, preferably 0.01-0.4%, further preferably 0.01-0.3%, further preferably 0.01-0.2%, and further preferably 0.05-0.2%, based on total mass of the beverage.

In some embodiments, the water has a mass percentage of 75-90%, based on total mass of the beverage.

In some embodiments, the raw materials further include a functional component.

In some embodiments, the functional component is selected from the group consisting of at least one of:
concentrated momordica grosvenori juice, natural menthol, concentrated liquorice juice, concentrated honeysuckle juice, chrysanthemum extract, kudzuvine root extract and loquat puree.

In some embodiments, the functional component has a mass percentage of 0.5-2%.

In some embodiments, the raw materials further include an acidity regulator.

In some embodiments, the acidity regulator is selected from the group consisting of at least one of:
tartaric acid, malic acid, citric acid and sodium citrate.

In some embodiments, the acidity regulator has a mass percentage of 0.1-5%.

In some embodiments, the raw materials further include a sweetening agent.

In some embodiments, the sweetening agent is selected from the group consisting of at least one of:
white granulated sugar, rock sugar, sucrose, maltose, corn syrup, glucose syrup, fructose, maltodextrin, polydextrose, xylitol, sorbitol, maltitol, erythritol, aspartame, acesulfame, sucralose, stevioside or neotame.

In some embodiments, the sweetening agent has a mass percentage of 0.5-5%.

In some embodiments, the raw materials further include an antioxidant.

In some embodiments, the antioxidant is selected from the group consisting of at least one of:
ascorbic acid, sodium erythorbate, vitamin C and vitamin E.

In some embodiments, the antioxidant has a mass percentage of 0.05-0.15%.

In some embodiments, the raw materials further include a preservative.

In some embodiments, the preservative is selected from the group consisting of at least one of:
sodium benzoate, sodium sorbate, and potassium sorbate.

In some embodiments, the preservative has a mass percentage of 0.01-0.05%.

In some embodiments, the raw materials further include an edible essence.

In some embodiments, the edible essence has a mass percentage of 0.5-5%.

### Embodiment 1

A beverage is prepared according to the following formula:
0.1wt% arecoline salt, 88wt% water, 1wt% concentrated honeysuckle juice, 1wt% concentrated liquorice juice, 2wt% citric acid, 2wt% sodium citrate, 3wt% stevioside, 0.05wt% vitamin C, 0.05wt% sodium sorbate and 2.8wt% edible essence.

Preparation method: the arecoline salt is fully mixed with water, concentrated honeysuckle juice, concentrated liquorice juice, citric acid, sodium citrate, stevioside, vitamin C, sodium sorbate and an edible essence to prepare a beverage.

The beverage prepared by the above preparation method retains various nutrients beneficial to human body in honeysuckle and liquorice. The beverage, rich in arecoline, areca polyphenols and other components, has a good taste, and has various effects of invigorating spleen and replenishing qi, clearing away heat and toxic materials, expelling phlegm to arrest coughing, relieving spasm and pain, and so on.

The written description discloses this application through embodiments, including the best mode, and also enables those skilled in the art to practice this application. The patentable scope of this application is defined by the claims, and may include other examples that occur to those skilled in the art. Such other examples are intended to be within the scope of the claims if they have structural elements that are not different from the literal language of the claims, or if they include equivalent structural elements without substantial differences from the literal language of the claims. All references cited herein are incorporated herein by reference to the extent that they do not cause inconsistency.

## Claims

1. An areca nut-flavored product, comprising an arecoline salt.

2. The product according to claim 1, wherein the arecoline salt is obtained by neutralizing arecoline with an complex acid.

3. The product according to claim 2, wherein the complex acid of the arecoline salt is selected from the group consisting of at least one of:
formic acid, acetic acid, propionic acid, butyric acid, isovaleric acid, 2-methylbutyric acid, valeric acid, 3-methylvaleric acid, 2-methyl-2-pentenoic acid, trans-2-hexenoic acid, hexanoic acid, heptanoic acid, octanoic acid, 2-nonenoic acid, decanoic acid, dodecanoic acid, tetradecanoic acid, hexadecanoic acid, octadecanoic acid, arachidic acid, salicylic acid, oxalic acid, citric acid, sorbic acid, tartaric acid, adipic acid, hydrochloric acid, hydrobromic acid, glycine, alanine, valine, leucine, isoleucine, methionine, phenylalanine, proline, tryptophan, serine, tyrosine, cysteine, methionine, asparagine, glutamine, threonine, aspartic acid, glutamic acid, lysine, arginine or histidine.

4. The product according to any one of claims 1 to 3, wherein the arecoline and the complex acid have a molar ratio of n; n has a value of 0<n≤10, preferably 0<n≤8, further preferably 0<n≤6, further preferably 0<n≤5, further preferably 0<n≤3, and further preferably 0<n≤2.

5. The product according to any one of claims 1 to 4, wherein the product is an aerosol generation substrate configured to be atomized to generate a smokable aerosol.

6. The product according to claim 5, wherein the arecoline salt has a mass percentage of 0.2-15%, preferably 1-15%, further preferably 2-15%, further preferably 2-10%, further preferably 2-5%, further preferably 2-4%, and more preferably 3-4%, based on total mass of the aerosol generation substrate.

7. The product according to claim 6, wherein the arecoline salt is arecoline hydrobromide and/or arecoline benzoate.

8. The product according to claim 7, wherein a method for preparing the arecoline salt comprises the following steps: extracting arecoline from areca nuts by using ethyl acetate as an extractant, adjusting a pH value of the extract to 7.5-8.5, taking an ethyl acetate layer, adding hydrobromic acid or benzoic acid to generate an arecoline salt crystal, and recrystallizing the arecoline salt crystal to obtain arecoline hydrobromide or arecoline benzoate.

9. The product according to claim 5, wherein the aerosol generation substrate further comprises a solvent and a flavoring agent.

10. The product according to claim 9, wherein the solvent is selected from the group consisting of at least one of:
propylene glycol, glycerol, polyethylene glycol 200, polyethylene glycol 400, dipropylene glycol ether, ethanol, water, triethyl citrate, triacetin, caprylic capric triglycerride, isopropyl alcohol, palm oil, peanut oil, corn oil or salad oil.

11. The product according to claim 10, wherein the solvent includes glycerol, and the glycerol in the aerosol generation substrate has a mass percentage of 0-90%.

12. The product according to claim 10, wherein the solvent includes propylene glycol, and the propylene glycol in the aerosol generation substrate has a mass percentage of 9-99.8%.

13. The product according to claim 9, wherein the flavoring agent is selected from the group consisting of at least one of:
nerol, trans-2-hexenol, linalool, benzyl alcohol, 1-hexanol, leaf alcohol, alpha-terpineol, citronellol, beta-phenylethanol, linalool oxide, geraniol, isoamyl alcohol, octanol, hexanol, decanol, cinnamyl alcohol, heptanol, eugenol, maltol, ethyl maltol, thymol, isoeugenol, 2-methylbutyric acid, malic acid, n-valeric acid, n-hexanoic acid, edible acetic acid, n-caprylic acid, strawberry acid, butyric acid, citric acid, propionic acid, 3-methylvaleric acid, isovaleric acid, isoamyl acetate, amyl formate, geranyl formate, butyl formate, benzyl formate, formicacidhexenester, gamma-decanolactone, delta-nonalactone, gamma-octalactone, gammaheptalactone, gamma-Undecalactone, delta-dodecalactone, benzaldehyde, strawberry aldehyde, cinnamaldehyde, furfural, citral, acetaldehyde, 3-methylthiopropanal, natural 3-mercapto-2-methylpentanal, isobutyraldehyde, trans-2-octenal, trans-2-nonenal, trans-2-decenal, trans,trans-2,4-heptadienal, 2,5-dimethylpyrazine, 2-acetylfuran, 2-ethyl-3(5 or 6)-dimethylpyrazine, 2,3,5,6-tetramethylpyrazine, 2,3,5-trimethylpyrazine, 2-acetylpyrazine, acetophenone, beta-ionone, damascenone No. 2 (Firmenich), butanedione, alpha-ionone, acetoin (acetyl methyl carbinol), acetoin (acetyl methyl carbinol), methyl heptenone, vanillin, ethyl vanillin, dihydrocoumarin, raspberry ketone, anisole, cedrol methyl ether, methyl-2-methyl-3-furyl disulfide, wintergreen oil, clove bud oil, 10-fold orange oil, Bois de rose oil, geranium oil, benzaldehyde, basil oil, ethyl vanillin, dihydrocoumarin, raspberry ketone, vanitrope, Tolu concrete, Peru concrete, oak extract, supercritical extract from espresso (water soluble), cocoa extract, coffee tincture, pandan leaf extract, vanilla extract, Labdanum concrete, orris oil or concrete, jasmine concrete, tree moss concrete (amber), tamarind extract, Zimbabwean tobacco extract, tobacco essence, burley tobacco extract, flue-cured tobacco absolute A, top note extract of flue-cured tobacco or top note extract of sun-cured tobacco.

14. The product according to any one of claims 5 to 13, wherein the aerosol generation substrate is a liquid phase configured to be atomized by any one of a heating atomizer, an ultrasonic atomizer, an air compression atomizer or a press-type spraying device to generate an aerosol.

15. The product according to any one of claims 5 to 13, wherein the aerosol generation substrate is a solid phase configured to generate a smokable aerosol when heated below an ignition point.

16. The product according to any one of claims 1 to 4, wherein the product is a filter capsule used in an aerosol-generating article.

17. The product according to any one of claims 1 to 4, wherein the product is a chewing gum.

18. The product according to any one of claims 1 to 4, wherein the product is a beverage.
